Europäisches Patentamt

**European Patent Office**

Office européen des brevets

(11) Publication number: **0 105 659**
**B1**

(12) **EUROPEAN PATENT SPECIFICATION**

(45) Date of publication of patent specification: **16.06.87**

(21) Application number: **83305525.4**

(22) Date of filing: **20.09.83**

(51) Int. Cl.⁴: **G 01 N 21/35,** G 01 N 21/75,
G 01 N 33/00

(54) **Carbon monoxide detectors.**

(30) Priority: **30.09.82 US 431430**

(43) Date of publication of application:
**18.04.84 Bulletin 84/16**

(45) Publication of the grant of the patent:
**16.06.87 Bulletin 87/25**

(84) Designated Contracting States:
**DE FR GB IT**

(56) References cited:
**FR-A-2 070 388**

**SIEMENS POWER ENGINEERING, vol. 5, no. 2,
March/April 1983, pages 50-52, Passau, DE; H.
MAYER et al.: "Immission measurements with
ultramat 4 to determine carbon monoxide
concentrations"**

(73) Proprietor: **THE BABCOCK & WILCOX
COMPANY**
**1010 Common Street P.O. Box 60035
New Orleans Louisiana 70160 (US)**

(72) Inventor: **Abromaitis, Andre Thomas
3012 Manor Drive
Northbrook Illinois 60062 (US)**
Inventor: **Keyes, Marion A. IV
120 Riverstone Drive
Chagrin Falls Ohio 44022 (US)**

(74) Representative: **Cotter, Ivan John et al
D. YOUNG & CO. 10 Staple Inn
London WC1V 7RD (GB)**

Courier Press, Leamington Spa, England.

## Description

This invention relates to carbon monoxide detectors.

Carbon monoxide (CO) is known to absorb infra-red radiation. CO detectors have taken advantage of this phenomenon to determine the difference between infra-red radiation absorption in a test cell containing a gas to be tested and a reference cell containing a fixed composition of gas, for example nitrogen. Since the background gas in the test and reference cells are different, inaccuracies may enter the detection operation.

The detection of carbon monoxide content is particularly important in flue gases generated in various industrial operations. One technique for detecting organic carbon as well as carbon monoxide in such industrial waste is disclosed in an article entitled "Continuous Method for Sampling Stack Gases for Total Carbon", G. G. Carels and R. C. Vincent, *Environmental Science and Technology*, Am. Chem. Soc., 1978, Vol. 12. This article mentions various techniques for detecting carbon monoxide, including the use of infra-red analysis.

According to the invention there is provided a carbon monoxide detector comprising a source of infra-red radiation, a measuring chamber for receiving a gas to be tested for carbon monoxide, and a reference chamber for receiving the gas, characterised in that:

a reaction chamber is connected between the measuring and reference chambers for converting carbon monoxide to carbon dioxide and for receiving the gas to be tested from the measuring chamber and supplying the gas which is free of carbon monoxide to the reference chamber;

the measuring and reference chambers have infra-red transparent means defining first and second infra-red radiation paths therethrough;

beam splitting means is associated with the source for directing infra-red radiation along the first and second paths;

an infra-red detector is provided for detecting alternately infra-red radiation of the first and second paths after the radiation has passed through the measuring and reference chambers, respectively; and

circuit means is connected to the detector for determining a difference between the infra-red radiation of the first and second paths absorbed in the measuring and reference chambers, which difference is a function of the carbon monoxide concentration of gas supplied to the measuring chamber.

A carbon monoxide detector forming a preferred embodiment of the invention and described hereinbelow utilises a single monochromatic infra-red source which produces infra-red (IR) light that is absorbed by carbon monoxide molecules. In addition, a single IR detector is utilised. Both source and detector are commutated between a measuring absorption chamber in the form of a tube and a reference absorbing chamber in the form of a tube to eliminate inaccuracies resulting from change in background concentrations, IR source intensity, or IR detector sensitivity. Carbon monoxide containing gas such as flue gas is supplied to the measuring absorbing tube. The gas is then supplied through a catalytic chamber which converts carbon monoxide (CO) to carbon dioxide ($CO_2$) and then to the reference absorbing tube or chamber. Since the flue gas itself is used as a reference gas, the interfering gases or background gases are the same for both tubes and therefore do not affect measurement accuracy.

The preferred CO detector incorporates the following features.

(i) There is a reflector in each of the first and second paths for reflecting IR radiation to a single detector.

(ii) A Pockel effect chopper is provided in each IR transparent means for alternately supplying IR radiation to the first and second paths.

(iii) The circuit means includes a log ratio circuit, the function thereof being:

$$C_1 = A_1 + A_2 I_n \frac{I_{OUT-2}}{I_{OUT-1}}$$

where $C_1$ is equal to the CO concentration in the measuring tube, $A_1$ and $A_2$ are calibration constants and $I_{OUT-1}$ and $I_{OUT-2}$ are the amounts of IR radiation from the first and second paths.

(iv) The detector is simple in design, rugged in construction and economical to manufacture.

The invention will now be further described, by way of illustrative and non-limiting example, with reference to the accompanying drawing, in which:

Figure 1 is a diagram illustrating a carbon monoxide detector embodying the invention; and

Figure 2 is a block diagram illustrating exemplary circuitry used in the detector of Figure 1 for determining carbon monoxide concentrations.

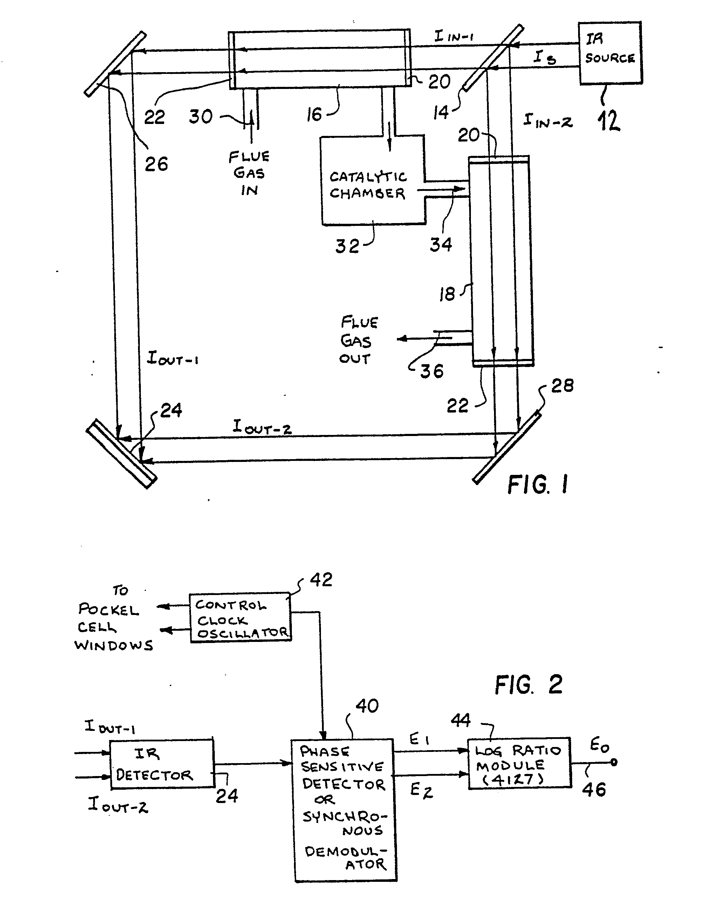

Figure 1 shows a monoxide (CO) detector that includes an infra-red (IR) source 12 which is a monochromatic source of IR radiation that is significantly absorbed by CO. The source 12 is used to generate IR radiation of an intensity $I_s$ which is separated into two equal components $I_{IN-1}$ and $I_{IN-2}$ by a beam splitter 14. The components $I_{IN-1}$ and $I_{IN-2}$ are alternately passed through first and second absorbing chambers or tubes 16, 18 by means of a Pockel effect chopper 20 in front of each tube and an IR transparent window 22 at the opposite end of each tube. IR radiation components of intensities $I_{OUT-1}$ and $I_{OUT-2}$ are then directed to a common IR detector 24 by means of IR reflectors 26 and 28. The intensities $I_{OUT-1}$ and $I_{OUT-2}$ are then measured alternately by means of a phase sensitive detector 40 (Figure 2) synchronised with the Pockel effect choppers 20.

In operation, flue gas or other CO-containing gas is supplied to the first (measuring) tube 16 over an inlet 30. The incoming flue gas must have sufficient available oxygen in it to convert all the CO to carbon dioxide ($CO_2$). This is usually available for low concentrations of CO. However, under some operating conditions, it may be desirable to add some ambient air to the incoming flue gas before it reaches the first absorbing tube 16. The gas then flows from the tube 16 to a catalytic chamber 32 where all the CO is converted to $CO_2$ in known fashion. CO-free gas with substantially the same background characteristic is then supplied to the second (reference) tube 18 over a connection 34, which gas finally flows out of the tube 18 via an outlet 36.

According to Beer's Law:

$$I_{OUT-1} = K_1 I_s (e^{-\mu L_1 (C_1 + B_1)}) \qquad (1)$$

$$I_{OUT-2} = K_2 I_s (e^{-\mu L_2 (C_2 + B_2)}) \qquad (2)$$

where:

$I_s$ = the intensity of the IR radiation from the source 12;

$K_1$ = a loss coefficient associated with the beam splitter 14, chopper 20, IR window 22, and reflector 26 for a first path through the tube 16 to the detector 24;

$K_2$ = a loss coefficient associated with the beam splitter 14, chopper 20, IR window 22, and reflector 28 for a second path through the tube 18 to the detector 24;

$\mu$ = an extinction coefficient for the particular wavelength being used;

$L_1$ = path length through the measuring absorbing tube 16;

$L_2$ = path length through the reference absorbing tube 18;

$C_1$ = CO concentration in the measuring absorbing tube 16;

$C_2$ = CO concentration in the reference absorbing tube 18;

$B_1$ = background gas concentration in the absorbing tube 16; and

$B_2$ = background gas concentration in the absorbung tube 18.

From equations (1) and (2), the following relationships can be derived:

$$\frac{I_{OUT-1}}{I_{OUT-2}} = \frac{K_1 I_s (e^{-\mu_1 L_1 (C_1 + B_1)})}{K_2 I_s (e^{-\mu_2 L_2 (C_2 + B_2)})} \qquad (3)$$

$$= \frac{K_1}{K_2} \frac{(e^{-\mu_1 L_1 C_1})(e^{-\mu_1 L_1 B_1})}{(e^{-\mu_2 L_1 C_2})(e^{-\mu_2 L_2 B_2})} \qquad (4)$$

Since $B_2 \simeq B_1$, $\mu_1 = \mu_2 = \mu$ and $L_1 = L_2$ by mechanical construction of the instrument, and since $C_2 = 0$, then:

$$\frac{I_{OUT-1}}{I_{OUT-2}} = \frac{K_1}{K_2} (e^{-\mu L C_1}) \qquad (5)$$

From Equation (5),

$$\frac{K_2 I_{OUT-1}}{K_1 I_{OUT-2}} = e^{-\mu L C_1} \qquad (6)$$

and

$$\ln \frac{K_2 I_{OUT-1}}{K_1 I_{OUT-2}} = - \mu L C_1 \qquad (7)$$

or:

$$C_1 = \frac{1}{\mu L} \ln \frac{K_1 I_{OUT-2}}{K_2 I_{OUT-1}} \qquad (8)$$

$$= \frac{1}{\mu L} (\ln \frac{K_1}{K_2} + \ln \frac{I_{OUT-2}}{I_{OUT-1}}) \qquad (9)$$

$$= A_1 + A_2 \ln \frac{I_{OUT-2}}{I_{OUT-1}} \qquad (10),$$

where $A_1$ and $A_2$ are treated as calibration constants in adjusting the gain and zero of the CO detector instrument.

Referring now to Figure 2, the IR detector 24, which alternately receives IR radiation along the first and second paths, supplies a signal to the phase sensitive detector (or synchronous demodulator) 40, which is controlled by a clock 42 that also functions to control the Pockel cell windows 20, 20. The demodulator 40 generates voltages $E_1$ and $E_2$ which are applied to a log ratio module 44. The voltages $E_1$ and $E_2$ are proportional to the intensities $I_{OUT-1}$ and $I_{OUT-2}$, respectively, of the IR radiation which travels along the first and second paths. The log ratio module 44 generates an output voltage $E_0$, on a line 46, which equals log $E_2/E_1$. This quantity, in turn, is directly proportional to the CO concentration $C_1$ and can be used to operate an indicator.

The Pockel effect described in *Reference Data for Radio Engineers,* Howard W. Sams and Company. The log ratio module 44 can be of the type designated 4127 manufactured by the Burr-Brown Research Corporation of Tuscon, Arizona, U.S.A.

**Claims**

1. A carbon monoxide detector comprising a source (12) of infra-red radiation, a measuring chamber (16) for receiving a gas to be tested for carbon monoxide, and a reference chamber (18) for receiving the gas, characterised in that:

a reaction chamber (32) is connected between the measuring and reference chambers (16, 18) for converting carbon monoxide to carbon dioxide and for receiving the gas to be tested from the measuring chamber (16) and supplying the gas which is free of carbon monoxide to the reference chamber (18);

the measuring and reference chambers (16, 18) have infra-red transparent means defining first and second infra-red radiation paths therethrough;

beam splitting means (14) is associated with the source (12) for directing infra-red radiation along the first and second paths;

an infra-red detector (24) is provided for detecting alternately infra-red radiation of the first and second paths after the radiation has passed through the measuring and reference chambers (16, 18), respectively; and

circuit means is connected to the detector (24) for determining a difference between the infra-red radiation of the first and second paths absorbed in the measuring and reference chambers (16, 18), which difference is a function of the carbon monoxide concentration of gas supplied to the measuring chamber (16).

2. A carbon monoxide detector according to claim 1, wherein the beam splitting means comprises a beam splitter (14) for receiving the infra-red radiation from the source (12) and supplying half of the radiation to the measuring chamber (16) and half of the radiation to the reference chamber (18).

3. A carbon monoxide detector according to claim 1 or claim 2, including an infra-red reflector (26, 28) in each of the first and second paths for reflecting the infra-red radiation to the infra-red detector (24).

4. A carbon monoxide detector according to claim 1, claim 2 or claim 3, wherein the reaction chamber (32) comprises a catalytic chamber for catalytically converting carbon monoxide to carbon dioxide.

5. A carbon monoxide detector according to any one of the preceding claims, wherein the circuit means comprises circuit elements for establishing the carbon monoxide concentration $(C_1)$ according to the function.

$$C_1 = A_1 + A_2 \ln \frac{I_{OUT-2}}{I_{OUT-1}},$$

where $A_1$ and $A_2$ are calibration constants, $I_{OUT-2}$ is the intensity of the infra-red radiation supplied to the detector (24) from the second path and $I_{OUT-1}$ is the intensity of the infra-red radiation supplied to the detector (24) from the first path.

6. A carbon monoxide detector according to any one of the preceding claims, wherein each of the infra-red transparent means includes a Pockel effect chopper (20) for transmitting infra-red radiation to the measuring and reference chambers (16, 18), respectively, at sequentially different times.

**Patentansprüche**

1. Kohlenmonoxiddetektor mit einer Infrarotstrahlungsquelle (12), einer Meßkammer (16) zur Aufnahme eines auf Kohlenmonoxid zu prüfenden Gases und einer Bezugskammer (18) zur Aufnahme des Gases, dadurch gekennzeichnet, daß:

eine Reaktionskammer (32) zwischen der Meß- und der Bezugskammer (16, 18) für die Umwandlung von Kohlenmonoxid in Kohlendioxid verbunden ist sowie zur Aufnahme des zu prüfenden Gases aus der Meßkammer (16) und Zuführung des an Kohlenmonoxid freien Gases zur Bezugskammer (18);

die Meß- und Bezugskammer (16, 18) für Infrarot transparente Einrichtungen haben unter Bildung erster und zweiter Infrarotstrahlungspfade durch die Kammer;

eine Strahlspalteinrichtung (14) der Quelle (12) zugeordnet ist, um Infrarotstrahlung längs des ersten und zweiten Pfades zu führen;

ein Infrarotdetektor (24) vorgesehen ist, um alternativ Infrarotstrahlung des ersten und zweiten Pfades zu erfassen, nachdem die Strahlung durch die Meß- bzw. Bezugskammer (16, 18) hindurchgegangen ist; und

ein Schaltmittel mit dem Detektor (24) verbunden ist, um eine Differenz zwischen der Infrarotstrahlung der ersten und zweiten Pfade zu bestimmen, welche in der Meß- und Bezugskammer (16, 18) absorbiert ist, wobei die Differenz eine Funktion der Kohlenmonoxidkonzentration von Gas ist, welches der Meßkammer (16) zugeführt wird.

2. Kohlenmonoxiddetektor nach Anspruch 1, wobei das Strahlenspaltmittel einen Strahlspalter (14) aufweist zur Aufnahme der Infrarotstrahlung aus der Quelle (12) und Zuführung der Hälfte der Strahlung zur Meßkammer (16) und der Hälfte der Strahlung zur Bezugskammer (18).

3. Kohlenmonoxiddetektor nach Anspruch 1 oder Anspruch 2, mit einem Infrarotreflektor (26, 28) sowohl in dem ersten als auch in dem zweiten Pfad zum Reflektieren der Infrarotstrahlung zum Infrarotdetektor (24).

4. Kohlenmonoxiddetektor nach Anspruch 1, Anspruch 2 oder Anspruch 3, wobei die Reaktionskammer (32) eine katalytische Kammer aufweist für die katalytische Umwandlung von Kohlenmonoxid in Kohlendioxid.

5. Kohlenmonoxiddetektor nach einem der vorhergehenden Ansprüche, wobei das Schaltmittel Schaltelemente aufweist zur Bildung der Kohlenmonoxidkonzentration $(C_1)$ nach der Funktion

$$C_1 = A_1 + A_2 \ln \frac{I_{OUT-2}}{I_{OUT-1}},$$

wobei $A_1$ und $A_2$ Kalibrierkonstanten sind, $I_{OUT-2}$ die Intensität der Infrarotstrahlung ist, die dem Detektor (24) aus dem zweiten Pfad zugeführt wird, und $I_{OUT-1}$ die Intensität der Infrarotstrahlung ist, die dem Detektor (24) aus dem ersten Pfad zugeführt ist.

6. Kohlenmonoxiddetektor nach einem der vorhergehenden Ansprüche, wobei jedes der für Infrarot transparenten Mittel einen Pockel-Effekt-Zerhacker (20) aufweist für den Durchlaß von Infrarotstrahlung zur Meß- bzw. Bezugskammer (16, 18) zu aufeinanderfolgend unterschiedlichen Zeiten.

## Revendications

1. Un détecteur de monoxyde de carbone comprenant une source (12) de rayonnement infrarouge, une chambre de mesure (16) destinée à recevoir un gaz à tester pour en déterminer la concentration en monoxyde de carbone, et une chambre de référence (18) destinée à recevoir le gaz, caractérisé en ce que:

une chambre de réaction (32) est branchée entre les chambres de mesure et de référence (16, 18) pour convertir le monoxyde de carbone en dioxyde de carbone, et pour recevoir le gaz à tester provenant de la chambre de mesure (16) et diriger vers la chambre de référence (18) le gaz qui est exempt de monoxyde de carbone;

les chambres de mesure et de référence (16, 18) comportent des moyens transparents à l'infrarouge qui définissent des premier et second chemins pour le rayonnement infrarouge qui traversent les chambres;

des moyens de division de faisceau (14) sont associés à la source (12) pour diriger le rayonnement infrarouge vers les premier et second chemins;

un détecteur infrarouge (24) est prévu pour détecter alternativement le rayonnement infrarouge des premier et second chemins, aprés que le rayonnement a traversé respectivement les chambres de mesure et de référence (16, 18); et

un circuit est connecté au détecteur (24) pour déterminer la différence entre les rayonnements infrarouges des premier et second chemins qui sont absorbés dans les chambres de mesure et de référence (16, 18), cette différence étant fonction de la concentration en monoxyde de carbone du gaz qui est introduit dans la chambre de mesure (16).

2. Un détecteur de monoxyde de carbon selon la revendication 1, dans lequel les moyens de division de faisceau consistent en un diviseur de faisceau (14) destiné à recevoir le rayonnement infrarouge provenant de la source (12) et à diriger la moitié du rayonnement vers la chambre de mesure (16) et la moitié du rayonnement vers la chambre de référence (18).

3. Un détecteur de monoxyde de carbone selon la revendication 1 ou la revendication 2, comprenant un réflecteur infrarouge (26, 28) dans chacun des premier et second chemins, destiné à réflechir le rayonnement infrarouge vers le détecteur infrarouge (24).

4. Un détecteur de monoxyde de carbone selon la revendication 1, la revendication 2 ou la revendication 3, dans lequel la chambre de réaction (32) consiste en une chambre catalytique destinée à convertir de façon catalytique le monoxyde de carbone en dioxide de carbone.

5. Un détecteur de monoxyde de carbone selon l'une quelconque des revendications précédentes, dans lequel le circuit comprend des éléments de circuit destinés à déterminer la concentration en monoxyde de carbone ($C_1$) conformément à la fonction:

$$C_1 = A_1 + A_2 \ln \frac{I_{OUT-2}}{I_{OUT-1}} \, ,$$

dans laquelle $A_1$ et $A_2$ sont des constantes d'étalonnage, $I_{OUT-2}$ est l'intensité du rayonnement infrarouge qui est applique au détecteur (24) à partir du second chemin, et $I_{OUT-1}$ est l'intensité du rayonnement infrarouge qui est appliqué au détecteur (24) à partir du premier chemin.

6. Un détecteur de monoxyde de carbone selon l'une quelconque des revendications précédentes, dans lequel chacun des moyens transparents à l'infrarouge comprend un dispositif de découpage à effet Pockels (20) destiné à transmettre le rayonnement infrarouge respectivement vers la chambre de mesure et la chambre de référence (16, 18) pendant des périodes successives différentes.

FIG. 1

FIG. 2